# EUROPEAN PATENT SPECIFICATION

(11) **EP 2 323 540 B1**
(45) Date of publication and mention of the grant of the patent: **14.11.2012**
(21) Application number: 09751365.9
(22) Date of filing: 19.05.2009
(51) Int. Cl.: A61B 1/00, A61B 1/018

(54) **INTEGRATED LOCKING DEVICE WITH FLUID CONTROL**
INTEGRIERTE VERSCHLUSSVORRICHTUNG MIT FLUIDKONTROLLE
DISPOSITIF DE VERROUILLAGE INTÉGRÉ AVEC RÉGULATION DE FLUIDE

(30) Priority: 19.05.2008 US 54413 P; 18.05.2009 US 467968
(43) Date of publication of application: 25.05.2011
(73) Proprietor: Boston Scientific Scimed, Inc., Maple Grove, MN 55311 (US)
(72) Inventor: WELDON, James, M., Newton MA 02459 (US); BYERS, Ransom, H., Mountain View CA 94040 (US); DILLON, Alexander, E., Cambridge MA 02139 (US); KOCHEM, Thomas, C., Belmont MA 02478 (US); RICE, Daniel, W., Gettysburg PA 17325 (US); SCHMIDT, Allison, L., Dothan AL 36305 (US); NIX, Lilly, Reston VA 20191 (US); CLARK, Lara, Salem VA 24153 (US); DONOHOO, Pearl, E., Cuyahoga Falls OH 44221 (US); HOLLETT, Andrew, K., West Bend WI 53095 (US); LITTLE, Chandra, Nashua NH 03062 (US)
(74) Representative: Vossius & Partner
(86) International application number: PCT/US2009/044493
(87) International publication number: WO 2009/143137

(56) References cited:
- US-A1- 2004 162 465
- US-A1- 2005 165 277
- US-A1- 2006 195 117

## Description

### FIELD OF THE INVENTION

The present invention pertains to endoscopes, endoscope assemblies, guidetubes, introducers, and instrument caps for endoscopes, guidetubes, and introducers. More particularly, the present invention pertains to biopsy caps for an access port of an endoscope.

### BACKGROUND

A wide variety of endoscope assemblies and biopsy caps have been developed. Of the known endoscope assemblies and biopsy caps, each has certain advantages and disadvantages. There is an ongoing need to provide alternative endoscope assemblies and biopsy caps as well as methods for making and using the same.

US 2006/195117 relates to a wire guide holder having a wire guide deflector. The wire guide holder is provided with a securing portion and a deflecting portion. The securing portion is provided with protrusions and/or grooves for securing a distal portion of a wire guide against longitudinal movement. The deflecting portion is configured to direct the proximal end of a wire guide into an unobtrusive position. A frictional element may be provided to prevent the wire guide from inadvertently sliding or falling out of the deflecting portion. The wire guide holder may be attached to a medical scope or a bite block. The wire guide holder may also be provided with a seal.

US 2004/162465 discloses a locking device for maintaining an elongated member at a selected place within a body lumen. The locking device comprises a substantially rigid body including an attachment portion adapted to be coupled to a distal portion of a medical instrument. When in an operative position, the distal portion of the medical instrument is received within the body lumen and an angled head coupled to the substantially rigid body. The angled head is configured to overlie an access port of the medical device when the attachment portion is coupled to the distal portion of the medical device in a predetermined configuration. A plurality of locking features extend from the angled head to immobilize a section of the elongated member relative to the medical device and a locking arm extends from the locking device so that, when the attachment portion is coupled to the medical device, the locking arm extends between the access port and the locking features to guide the elongated member from the access port to the locking features along a desired path.

### BRIEF SUMMARY

The invention relates to biopsy caps according to the claims.

The invention provides design, material, and manufacturing method alternatives for endoscope assemblies and biopsy caps as well as provides methods for making and using endoscope assemblies and biopsy caps.

An example endoscope assembly may include an endoscope having a channel formed therein and a port that provides access to the channel. A cap may be coupled to the port. The cap may include a base having a securing member for securing the cap to the port. The cap may also include an outer shell, a locking member coupled to the shell, an inner seal member disposed within the shell, and one or more openings extending through the cap and into the channel. A fluid control region may be defined within the shell.

An example endoscope biopsy cap may include an outer shell having an opening formed therein and a base. A fluid control region may be defined within the shell. A securing member may be disposed on the base for securing the base to a port on an endoscope. A locking member may be coupled to the shell for securing the position of a medical device disposed in the opening. A seal member may be disposed within the shell.

Another example endoscope biopsy cap may include an outer shell, a securing member coupled to the shell, a locking member coupled to the shell, a seal member disposed within the shell, and means for controlling fluid disposed within the shell.

An illustrative example method for using an endoscope may include providing an endoscope having a channel formed therein and a port that provides access to the channel, disposing the endoscope within a body lumen of a patient, and disposing a cap on the port. The cap may include an outer shell having an opening formed therein and a base, a securing member disposed on the base for securing the base to a port on an endoscope, a locking member coupled to the shell for securing the position of a medical device disposed in the opening, and a seal member disposed within the shell. A fluid control region may be defined within the shell.

The above summary of some embodiments is not intended to describe each disclosed embodiment or every implementation of the present invention. The Figures, and Detailed Description, which follow, more particularly exemplify these embodiments.

### BRIEF DESCRIPTION OF THE DRAWINGS

The invention may be more completely understood in consideration of the following detailed description of various embodiments of the invention in connection with the accompanying drawings, in which:
FIG. 1 is a perspective view of an example endoscope assembly;
FIG. 2 is an exploded view of a portion of the example endoscope assembly shown in FIG. 1 illustrating a biopsy cap;
FIG. 3 is a partial cross-sectional view of an example biopsy cap;
FIG. 4 is a bottom view of the example biopsy cap illustrated in FIG. 3;
FIG. 5A is a cross-sectional view of another example biopsy cap;
FIG. 5B is a plan view of an example locking member for use with a biopsy cap;
FIG. 6A is a perspective view of an example biopsy cap;
FIG. 6B is an alternative view of the example biopsy cap of FIG. 6A;
FIG. 6C is another alternative view of the example biopsy cap of FIG. 6A;
FIG. 6D is a cross-sectional view of the example biopsy cap of FIG. 6A shown disposed on an endoscope port;
FIG. 6E is a perspective view of a foam seal insert included in the biopsy cap of FIG. 6D;
FIG. 7 is a perspective view of an example securing member;
FIG. 8 is a perspective view of another example securing member;
FIG. 9 is a perspective view of another example securing member;
FIG. 10A is a perspective view of another example securing member in a first configuration;
FIG. 10B is a perspective view of the example securing member illustrated in FIG. 10A in a second configuration;
FIG. 11 is a perspective view of another example securing member;
FIG. 12 is a perspective view of another example securing member;
FIG. 13A is a perspective view of an example locking member in a first configuration;
FIG. 13B is a perspective view of the example locking member illustrated in FIG. 13A in a second configuration;
FIG. 14 is a perspective view of another example locking member;
FIG. 15A is a perspective view of another example locking member;
FIG. 15B is a perspective view of an alternative locking member to that depicted in FIG. 15A;
FIG. 15C is a perspective view of the alternative locking member of FIG. 15B showing further details;
FIG. 16 is a perspective view of another example locking member;
FIG. 17 is a perspective view of another example locking member;
FIG. 18 is a perspective view of another example locking member;
FIG. 19A is a perspective view of another example locking member in a first configuration;
FIG. 19B is a perspective view of the example locking member illustrated in FIG. 19A in a second configuration;
FIG. 20A is a perspective view of another example locking member in a first configuration;
FIG. 20B is a perspective view of the example locking member illustrated in FIG. 20A in a second configuration;
FIG. 21A is a perspective view of another example locking member in a first configuration;
FIG. 21B is a perspective view of the example locking member illustrated in FIG. 21A in a second configuration; and
FIG. 22 is a perspective view of another example locking member.

While the invention is amenable to various modifications and alternative forms, specifics thereof have been shown by way of example in the drawings and will be described in detail.

### DETAILED DESCRIPTION

For the following defined terms, these definitions shall be applied unless a different definition is given in the claims or elsewhere in this specification.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following detailed description should be read with reference to the drawings in which similar elements in different drawings are numbered the same. The drawings, which are not necessarily to scale, depict illustrative embodiments and are not intended to limit the scope of the invention.

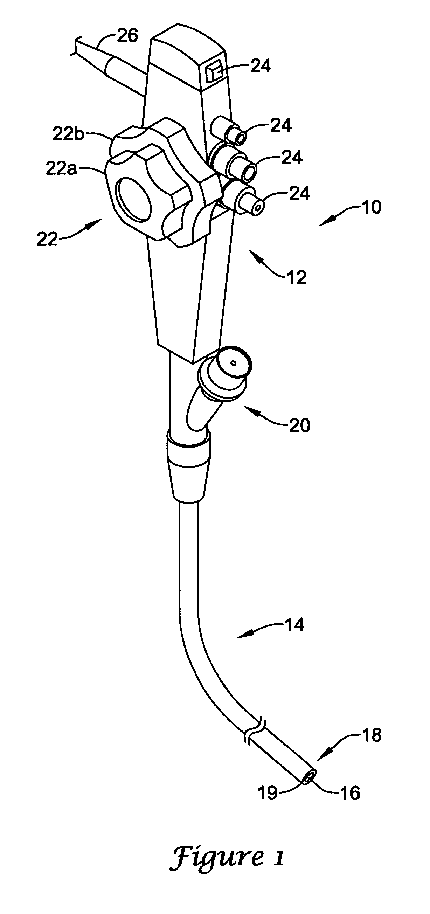

An example endoscope and/or endoscope assembly 10 is illustrated in FIG. 1. Endoscope 10 may be any of a number of types of endoscopes or related medical devices usually identified by the particular anatomy desired to be reached. For example, endoscope 10 may be a bronchoscope, colonoscope, duodenoscope, esophagoscope, guidetubes, introducers (without or without vision or visualization capabilities), or any other type of endoscope or related medical device. Endoscope 10 may include a handpiece 12 and an elongate shaft 14 extending distally from handpiece 12 to a distal tip 18. Shaft 14 may include a lumen defining a working channel 16 extending through shaft 14 from a distal end 19 near distal tip 18 of shaft 14 to an access port 20 that may be positioned in handpiece 12 or another portion of endoscope 10. Although endoscope 10 is depicted with a single working channel in FIG. 1, it can be appreciated that in other embodiments, endoscope 10 may include multiple working channels, as desired.

Handpiece 12 may include one or a plurality of controls 22, such as rotating knobs, which may be used to control movement of distal tip 18 of shaft 14 during operation. For example, a first rotating knob 22a may control up and down movement or deflection of distal tip 18 of shaft 14, while a second rotating knob 22b may control side-to-side movement or deflection of distal tip 18 of shaft 14. Handpiece 12 may also include one or a plurality of buttons 24, which may be used to activate suction or deliver fluid such as air, saline and/or water, etc. through a lumen of the endoscope 10 or perform other functions as desired. Additionally, handpiece 12 may include an optical cable 26 connected to an external light source (not shown).

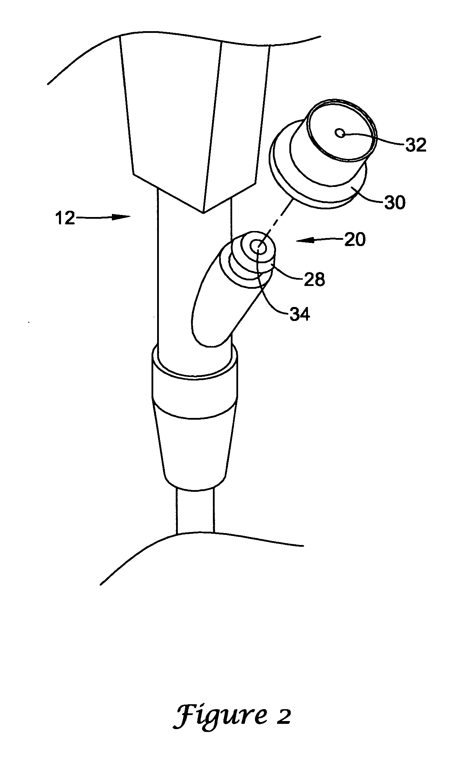

Turning now to FIG. 2, here access port 20 of handpiece 12, which provides access to working channel 16 of endoscope 10, is illustrated. Access port 20, which may extend from the side of endoscope 10 or at another location, may include a coupling portion 28 for coupling a cap 30 to access port 20. Cap 30, which may be removably attached or permanently attached to access port 20, may provide access for inserting and/or advancing an endoscopic device through working channel 16 of endoscope 10.

Caps like cap 30, which may be termed "biopsy caps", are often designed with several functions in mind. For example, cap 30 may form a fluid/air barrier to working channel 16 that may help control insufflation and bile fluid egress therefrom that later have the potential to spill onto the clinician's hands and/or the floor thereby interfering with the intervention and/or become a biohazard. In addition, cap 30 may have an opening 32 extending therethrough. Opening 32 may be in fluid communication with working channel 16 and it may reduce the size of the opening 34 of working channel 16, for example, to accommodate an endoscopic device or instrument. Thus, caps like cap 30 may be much like an adapter in that it forms a physical transition at opening 34 of working channel 16 (or other instrument channels or access points) so that it transitions to a size more closely to that of the device to be inserted into working channel 16. Some additional discussion regarding biopsy caps can be found in U.S. Patent Application Pub. Nos. US20070293719AI, US20070244356A1, and US20070238928A1.

A number of additional biopsy caps are contemplated that incorporate at least some of the desirable features of biopsy caps as well as have other desirable characteristics. The forgoing discussion discloses some of the embodiments of caps that are contemplated. These caps may include an active seal. For the purposes of this disclosure, an active seal is a seal that when actuated by a user seals endoscope 10 at port 20 so as to prevent the leakage of bodily fluids and/or air. In addition, by virtue of being "active", the caps disclosed herein are configured to be selectively activated so as to seal off endoscope 10 at port 20 at any appropriate time during an intervention and, conversely, may also be configured to be "unlocked" or "unsealed" at the desired time by a clinician.

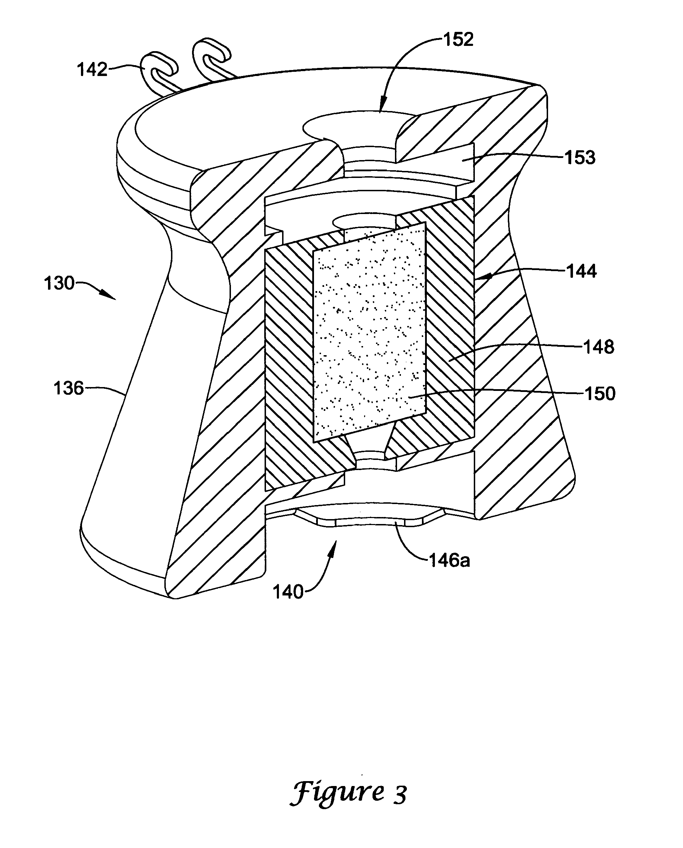

Turning now to the remaining figures, FIG. 3 is a partial cross-sectional side view of biopsy cap 130, which may be similar in form and function to other caps disclosed herein. Here it can be seen that cap 130 may include an outer shell 136, a securing member 140 that may help to secure cap 130 to port 20, one or more locking members 142 coupled to shell 136, and an inner seal member 144 disposed within shell 136. Outer shell 136 may take a number of different shapes and forms. In general, however, outer shell 136 may be made from a relatively rigid or hard polymer/plastic, a metal or metal alloy, a ceramic, and the like, or combinations thereof and may take a form resembling an exoskeleton or protective covering over the more delicate interior (e.g., seal member 144). In addition, by virtue of forming shell 136 from a relatively rigid material, a number of accessories to and/or structural components of cap 130 may be secured to or integrally formed with shell 136. For example, securing member 140 and/or locking members 142 may be secured to or integrally formed with shell 136.

Shell 136 may have one or more apertures 152 formed therein. Aperture 152, for example, may be disposed on a top surface or surface that is opposite securing member 140 and may extend all the way through cap 130 including, for example, through seal member 144 and securing member 140. Although aperture 152 is illustrated on the top of shell 136, it can be appreciate that essentially any other suitable portion of shell 136 may include apertures 152 including the sides or side surfaces of shell 136. Aperture 152 may be the entrance point or otherwise define one or more openings that extend through cap 130 into channel 16 when cap 130 is seated on port 20. Thus, aperture 152 may form the exterior opening in cap 130 where other medical devices (e.g., guidewires, catheters, etc.) can be passed through so as to gain access to working channel 16.

To ease the ability of a user to pass a medical device through aperture 152, apertures 152 may have a chamfered or beveled edge, which may function like a funnel to guide the medical device into aperture. In addition to the funneling function that may be realized by the inclusion of beveled aperture 152, aperture 152 may also provide cap 130 with a number of additional desired characteristics. For example, because aperture 152 is formed in the relatively rigid shell 136 and because they are generally positioned a distance away from port 20, aperture 152 and/or shell 136 may also function as a strain relief that may relieve strain that might otherwise be applied to endoscope 10 (e.g., at port 20), for example, during device exchanges or transfers. Thus, the shear stress that may be generated during device exchanges can be shifted away from endoscope 10 (and sealing member 144), which may improve the ability of cap 130 to maintain a good seal at port 20.

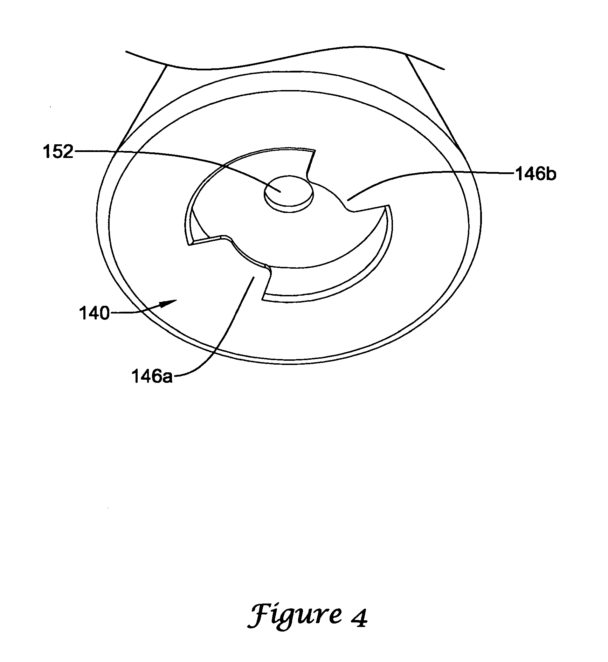

Securing member 140 may be disposed on a bottom surface of cap 130 as illustrated in FIGS. 3 and 4. Securing member 140 may take any number of a wide array of forms including those disclosed herein. For example, securing member 140 may include a pair of tabs 146a/146b, which may snap onto or otherwise secure to port 20. Securing tabs 146a/146b onto port 20 may include, for example, snapping tabs 146a/146b onto a narrowed ring or portion of port 20. This may include snapping tabs 146a/146b onto port from a top or end region of port 20 (e.g., "from above"). In addition, a portion of shell 136 may include a cutout or notch (not shown, but the cutout may be formed along a portion of cap 130 adjacent where the cross-section is taken in FIG. 3, for example) that may provide some structural relief for securing member 140 and that may allow tabs 146a/146b to have greater flexibility when securing cap 130 to port 20. The precise form of securing member 140 and/or tabs 146a/146b may vary. For example, a different number of tabs may be utilized, differently shaped tabs may be utilized or a different securing system altogether may be utilized for securing cap 130 to port 20. Examples of some of the various alternative securing members 140 contemplated can be found below.

Locking members 142 may be generally disposed adjacent the top surface of cap 130 and they may be used to secure and/or hold the position of a device (e.g., a guidewire, catheter, etc.) extending through cap 130 into channel 16. However, locking members 142 may be disposed on any suitable surface of cap 130 and/or shell 136. Locking members 142 may also be integrally formed with shell 136. In addition to holding the position of a device, locking members 142 may also tend to guide these devices away from the center of cap 130 so that other device may gain access to channel 16 via cap 130. Locking members 142 may include one or more bends or "hooks" formed therein that a medical device may be wrapped around or pressed against to hold its position. The number of locking members 142 may vary. In some embodiments, one locking member 142 is utilized. In other embodiments, two, three, four, five, six, or more locking members 142 are utilized. In addition, the precise form of locking members 142 may also vary. Examples of some of the various alternative locking members 142 contemplated can be found below.

Seal member 144 may include an outer portion 148 and an inner portion 150. In at least some embodiments, outer portion 148 may be made from a sealing material such as, for example, an elastomer. Other materials, of course, are contemplated. The elastomeric outer portion 148 may be chamfered adjacent aperture 152 so as to guide or funnel devices therethrough. Outer portion 148 may extend laterally to the edges of shell 136. This may help to prevent or reduce the amount of fluids that may migrate into and out from cap 130. Outer portion 148 may also extend to the top of shell 136 and may form a strain relief. Alternatively, a gap 153 may be formed between the top of outer portion 148 and the top of the interior of shell 136. Gap 153 may provide an area for fluids to collect that may escape seal member 144 and that may otherwise "splash" during, for example, device removal or exchange.

Inner portion 150 may comprise a soft material such as a plastic or foam that may be suitable for sealing about a medical device extending therethrough. The precise form and materials for inner portion 150 may vary. For example, inner portion 150 may include a pliable or formable polymer that may be absorbent. In some embodiments, inner portion 150 may include those materials used for similar structures disclosed in U.S. Patent No. 6,663,598. In at least some embodiments, inner portion 150 may substantially fill the interior of outer portion 148. Alternatively, a portion of the interior of outer portion 148 may lack inner portion 150 and may be used, for example, to hold bodily fluids that may escape from port 20.

In some embodiments, inner portion 150 may include a fluid, gel or gel-like material that is capable of both sealing around devices extending therethrough and absorbing fluids. For example, inner portion 150 may include a petroleum jelly or similar substance such as VASELINE® petroleum jelly and/or KY® jelly. Alternatively, inner portion 150 may include a hygroscopic material that may expand upon absorption of fluids and may place additional sealing force on devices extending therethrough. Accordingly, inner portion 150 may help control bodily or other fluids that may be disposed in cap 130 and thus may be understood to be a fluid control region or portion of cap 130.

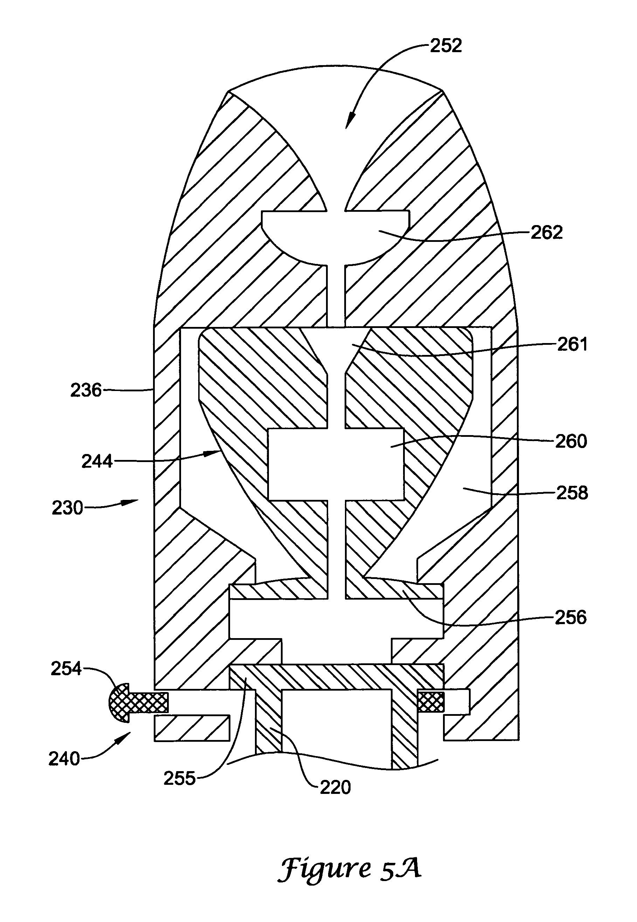

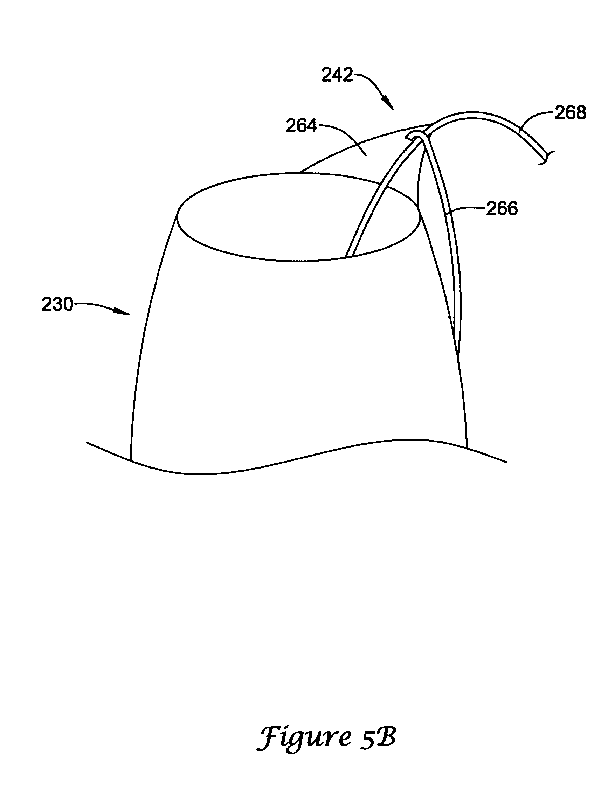

FIG. 5A illustrates another example cap 230 that may be similar in form and function to other caps disclosed herein. Cap 230 may include shell 236, securing member 240, seal member 244, and aperture 252 extending therethrough. Aperture 252 may be chamfered or funnel-shaped to help facilitate device insertion into a chamfered or funnel-shaped portion 261 of seal member 244. At least some of the features or components of cap 230 may be similar in form and function to similar structures in other caps disclosed herein. Cap 230 may also include locking member 242, which is omitted from FIG. 5A for simplicity purposes and is illustrated in FIG. 5B.

Securing member 240 may include a slidable button lock 254 that may be actuated to lock or unlock cap 230 from port 220. To facilitate locking, port 220 may include a flange 255 that button lock 254 may catch upon when locking securing member 240. Furthermore, because the position of button lock 254 may be readily viewed by a user, button lock 254 may provide visual confirmation of whether or not cap 230 is satisfactorily locked onto port 220. Other securing members, including any of those disclosed herein, may be substituted for securing member 240 without departing from the spirit of the invention.

Seal member 244 may be seated within shell 236 and secured to shell 236 (e.g., along the interior wall of shell 236) at a plurality of points. However, gaps may be left between seal member 244 and shell including, for example, gap 258. This arrangement may be desirable because it may allow fluid that might otherwise migrate up through seal member 244 to flow into gap 258 and onto a valve or gasket portion 256 of seal member 244. Accordingly, when suction is applied through the endoscope (e.g., via another aspiration tube or lumen so as to create suction on the working channel of the endoscope), valve portion 256 may deform downward (e.g., toward port 220), allowing any fluids collected thereon to flow back into port 220. Thus, gap 258 may define a fluid control region where bodily or other fluids can be housed until a clinician can remove it (e.g., via aspiration, suction, etc.) or otherwise deal appropriately with it.

Cap 230 may also include one or more additional fluid control regions. These regions may also help to collect and/or hold bodily fluids that may accumulate within cap 230. For example, a fluid control chamber 260 may be defined within seal member 244. Chamber 260 may take the form of an opening or widening of seal member 244 that is generally disposed within seal member 244. Thus, fluids that may escape from around a device that extends through seal member 244 may be collected in chamber 260. Another fluid control chamber 262 may be disposed within shell 236, for example, above seal member 244. Chamber 262 may collect fluids that escape from seal member 244 altogether. Chambers 260/262 may be designed to hold a volume of fluid until the time when it might be convenient for a clinician to dispose of the fluid. For example, aspiration and/or suction may be applied to the endoscope (e.g., on the working channel) so that suction pulls downward on cap 230. This may allow fluids that are collected in cap 230 to simply migrate back into the endoscope.

In addition to the fluid control regions illustrated in FIG. 5, a number of additional or alternative fluid control regions may also be defined in cap 230 as well as other caps disclosed herein. For example, one or more channels or chambers may be formed in the wall of shell 236. Access to these chambers may include one or more openings that feed into the chambers. Accordingly, fluid that might otherwise collect within cap 230 may migrate into the chamber via the apertures. Numerous other fluid control regions are contemplated including various chambers, channels, reservoirs, openings, valves, and the like that may be formed in or around cap 230.

FIG. 5B illustrates locking member 242, which may be positioned near the top of cap 230. The form of locking member 242 may vary considerably. For example, locking member 242 may include a wing or flap member 264 and a hook member 266. Wing member 264 may funnel or otherwise direct a device 268 (e.g., a guidewire, catheter, etc.) toward hook member 266. In addition, wing member 264 may be biased to press against hook member 266 so that wing member 264 may also hold device 268 against hook member 266. Examples of some of the various alternative locking members that may be utilized with cap 230 or other caps disclosed herein are contemplated and can be found below.

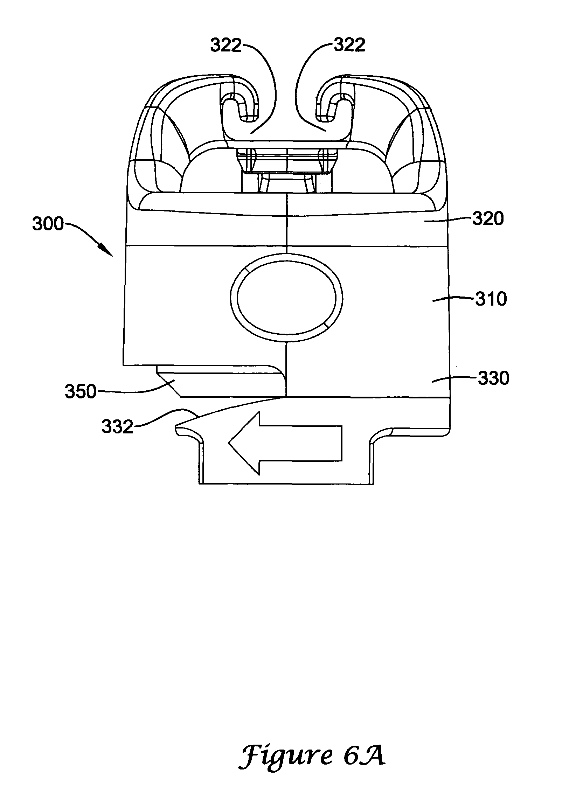

An alternative biopsy cap design and details thereof are depicted in FIGS. 6A-6E. The design incorporates certain features of biopsy caps previously discussed but with certain variations in the functional parts. FIG. 6A is a perspective view of this alternative embodiment of a biopsy cap 300. The biopsy cap 300 includes a shell 310 having an upper portion 320 and a lower portion 330. The shell 310 can be formed of a single piece or, as illustrated in FIG. 6A, upper portion 320 of shell 310 may be a separate piece from lower portion 330 of shell 310. Upon assembly, the upper portion 320 and lower portion 310 may be held together by any suitable means, such as interlocking tabs, a snap fit or an adhesive. As discussed in detail below, a seal member 350 is disposed within the shell 310.

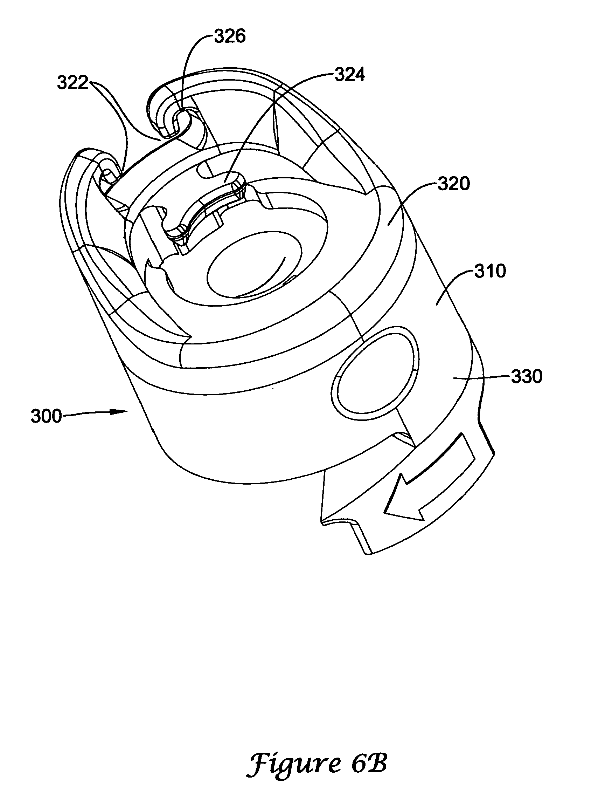

The upper portion 320 of shell 310 preferably has integrally formed thereon one or more locking members 322. As with other embodiments disclosed herein, locking members 322 are used to secure a guidewire, catheter or other instrument at a fixed longitudinal position relative to the endoscope during a procedure. As better illustrated in FIG. 6B, which is an alternative view of the biopsy cap 300 of FIG. 6A, each locking member includes a slotted portion 324 and hook portion 326. The slotted portion 324 and hook portion 326 cooperate to retain an instrument extending there through in a fixed position.

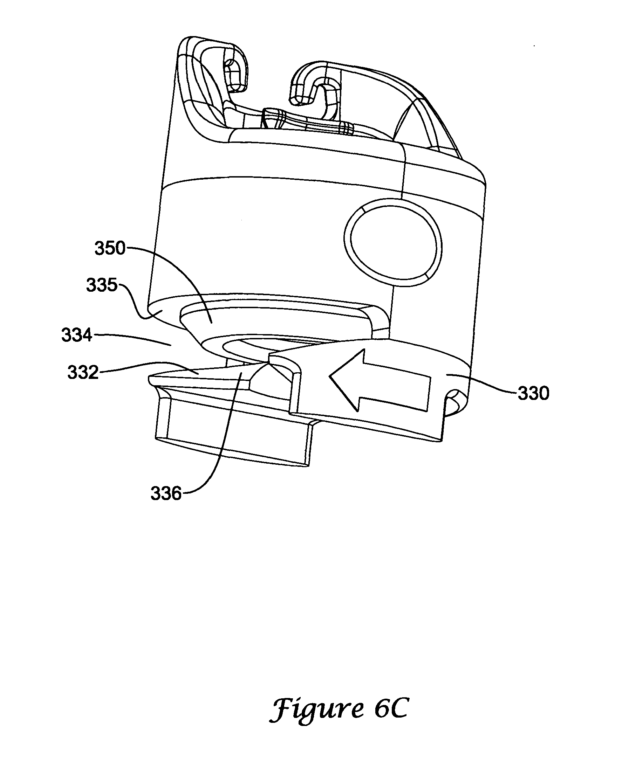

The lower portion 330 of shell 310 preferably has integrally formed thereon a base incorporating a securing member 332. As with other embodiments disclosed herein, securing member 332 functions to secure the biopsy cap to the entrance or port of the working channel of the endoscope. The features of this exemplary securing member 332 are better illustrated in FIG. 6C, which is an alternative view of the biopsy cap 300 of FIG. 6A. The lower portion 330 has a mouth 334 formed therein for receiving the connecting portion of the port on the working channel of an endoscope. The mouth 334 is bounded on the upper side by the seal member 350 which extends into the mouth 334 to sealingly engage the endoscope port. The mouth 334 is bounded on the lower side by a ramped surface 336. The ramped surface assists in lateral placement of the endoscope port into the mouth 334 from the upper surface 335 of mouth 334 so that it is easily aligned and pushed into sealing engagement with the seal member 336. The ramped surface 336 can be formed with a shoulder or other suitable structure that yields an audible snap when the biopsy cap 300 is properly placed on the endoscope port. Alternatively, the seal member 350 can include a cavity at it lower end into which the endoscope port fits. With this design, the user can hear or feel the biopsy cap snapping into place when the seal member properly engages the endoscope port.

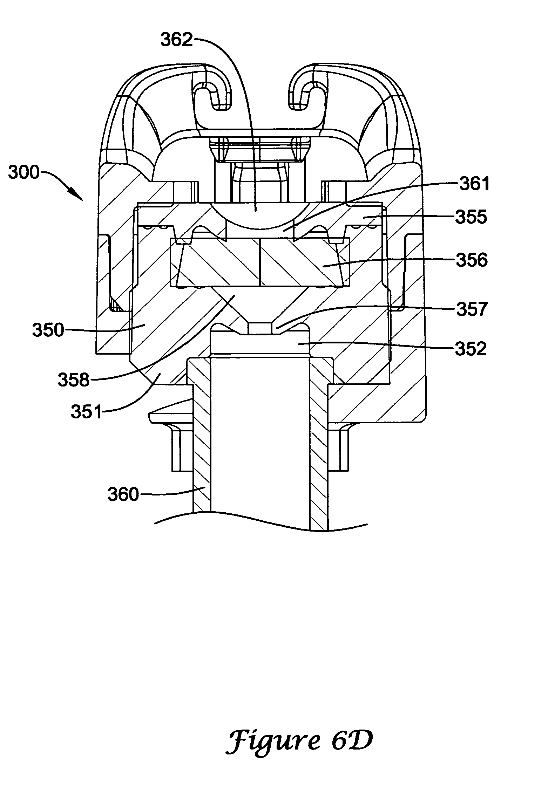

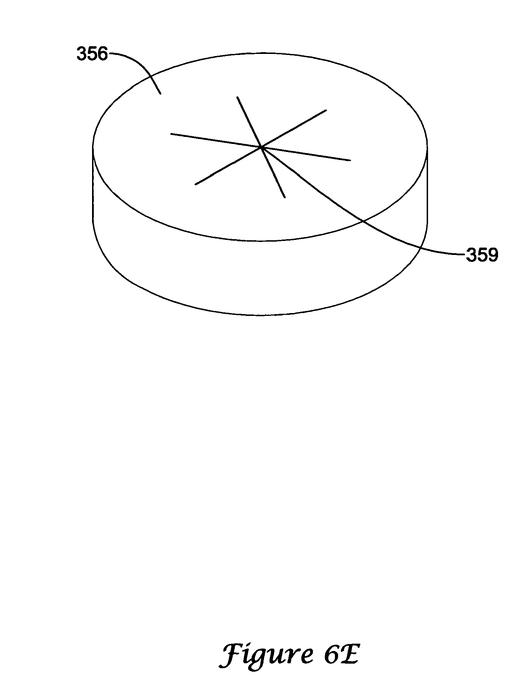

The function of the seal member 350 and its structural details are best shown in FIGS. 6D and 6E. FIG. 6D is a cross-sectional view of the biopsy cap 300 positioned on the port 360 of an endoscope (not shown). Further, in this exemplary embodiment, the seal member 350 includes a lower portion 351, an upper portion 355 and a foam insert member 356. As shown, the port 360 sealingly engages the seal member 350. The lower portion 351 of seal member 350 includes a cavity 352 into which the port 360 fits when properly positioned. The lower portion 351 of seal member 350 is made from a resilient or elastomeric material so that it deflects to a degree as the port 360 is moved laterally into mouth 334. As deflected, the seal member 350 exerts force on the port to sealingly engage the port 360 and assists in keeping the biopsy cap in position during use. The lower portion 351 of seal member 350 also includes a lower seal 357 which engages a device passing there through. Above the lower seal 357, a fluid control region or cavity is formed. A foam insert member 356 fills a portion of this cavity 358.

The foam insert member 356 is depicted in perspective view in FIG. 6E. Foam insert member 356 is a generally cylindrical piece of foam having a plurality of slits 359 extending radially from the center and through the length of the foam insert member 356. The slits 359 allow passage of an instrument therethrough, and the deflected foam material forms a seal against the outer surface of the instrument.

The seal member 350 also includes an upper portion 355 which lies over and is attached to the upper surface of the lower portion 351 of seal member 350. The upper portion 355 encloses the top of the cavity 359. The upper portion 355 also includes a slit type seal 361 formed there through in an indented portion 362 of its surface. The indented portion 362 provides a thin layer which stretches when an instrument is inserted through the slit type seal 361. The seal member 350 in this exemplary embodiment therefore includes three seals including an uppers slit type seal, a foam insert seal and a lower seal. Further, a fluid control cavity is formed between the upper and lower seals.

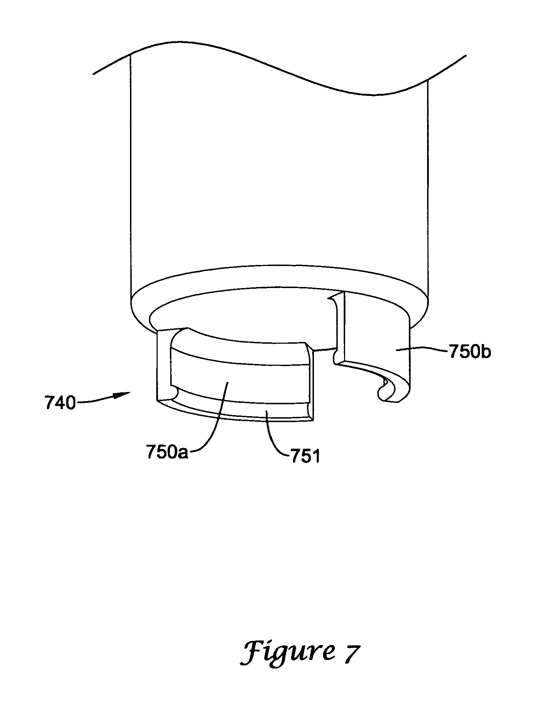

FIGS. 7-13 illustrate some examples of securing members that may be utilized in any of the biopsy caps disclosed herein. For example, FIG. 7 illustrates securing member 740, which may be similar to securing members 140/240 and/or other securing members disclosed herein. Securing member 740 may include a pair of locking tabs 750a/750b that may be fitted onto and secured to port 20. Tabs 750a/750b, which may be similar to tabs 150a/150b and/or 250a/250b, may be configured to snap onto port 20. In some embodiments, tabs 750a/750b may include a ridge 751. Ridge 751 may help to hold securing member 740 onto port 20 and/or mate with another ridge 751 or groove that may be formed on port 20.

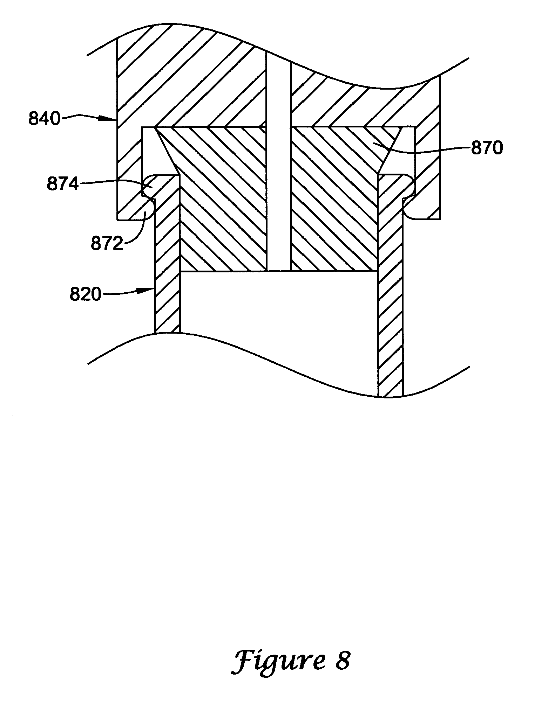

FIG. 8 illustrates another example securing member 840, which may be utilized in conjunction with any of the caps disclosed herein. Securing member 840 may include a plug or stopper 870 that may be configured to fit within port 820 in a manner similar to a cork or elastomeric stopper. Indeed, plug 870 may be made from similar materials including, for example, silicone, cork, an elastomer, rubber, and the like or other suitable sealing materials. In addition, securing member 840 may also include a ridge 872 that helps to hold securing member 840 in place, for example, adjacent a ridge 874 formed on the end of port 820. Alternatively, port 820 may include a groove (not shown) that is configured to mate with ridge 872.

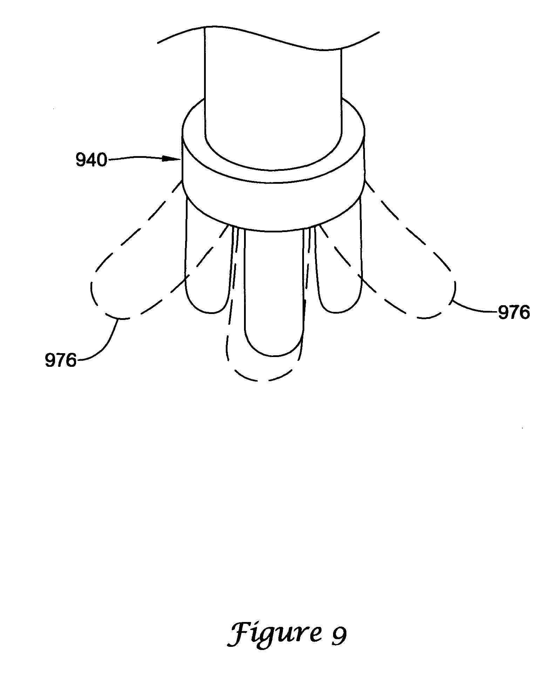

FIG. 9 illustrates another example securing member 940. Securing member 940 may include a plurality of leaf-like fingers 976. Fingers 976 may be configured to shift between a first or "open" position (depicted in phantom) and a second or "closed" position, the later being used to hold securing member 940 onto an access port. In some embodiments, fingers 976 may grasp onto or hold onto a side portion of the access port and/or the endoscope. Alternatively, fingers 976 may wrap around (e.g., the back side of) the access port and/or endoscope.

In at least some embodiments, fingers 976 are made from a deformable material that allows them to shift between the first and second positions and hold the desired shape/position. Leaf-like fingers 976 may be utilized alone as a securing member 940, as shown, or as a secondary attachment means by combining them with other securing members 940.

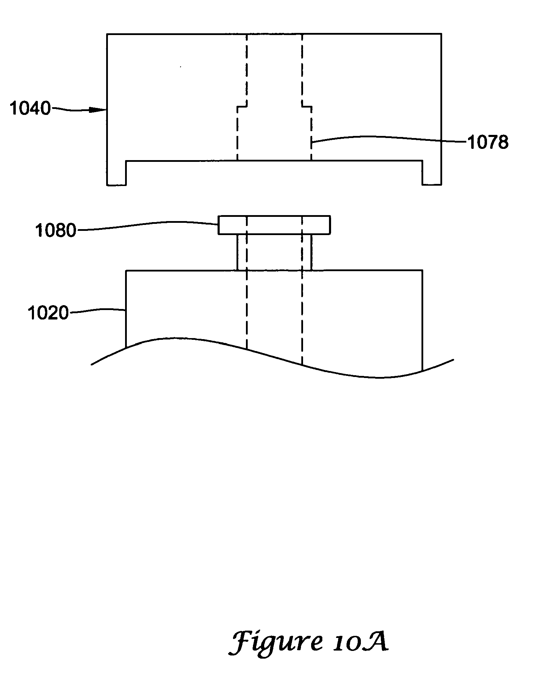

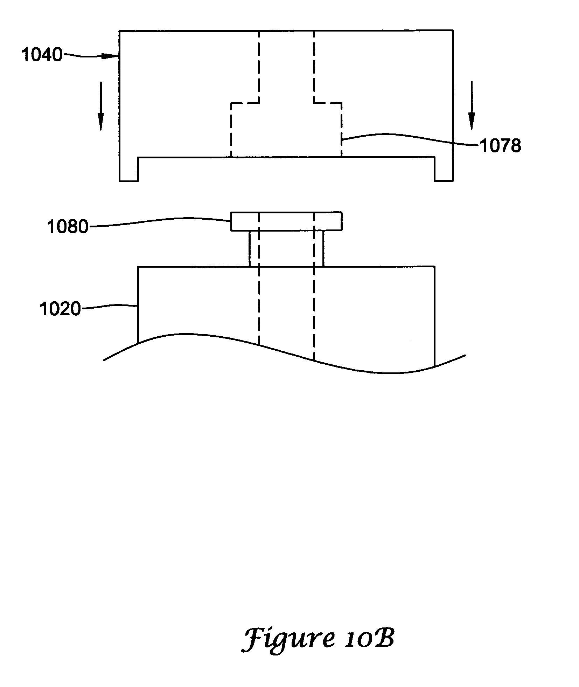

FIGS. 10A and 10B illustrate another example securing member 1040. Securing member 1040 may include an end with a deformable or flexible opening 1078 that is configured to shift between a narrowed configuration (illustrated in FIG. 10A) and an expanded configuration (illustrated in FIG. 10B). When in the expanded configuration, opening 1078 may be fitted over a portion of port 1020, for example, such as a flange or ridge 1080 on port 1020. Shifting may be accomplished by the nature of the material in which opening 1078 is formed. For example, the end of securing member 1040 may include a flexible polymer, an elastomer, silicone, rubber, and the like, or any other suitable material that may be resilient enough to undergo the necessary shifts in size. Once seated, opening 1078 may shift or partially shift back to the narrowed configuration and seal about port 1020.

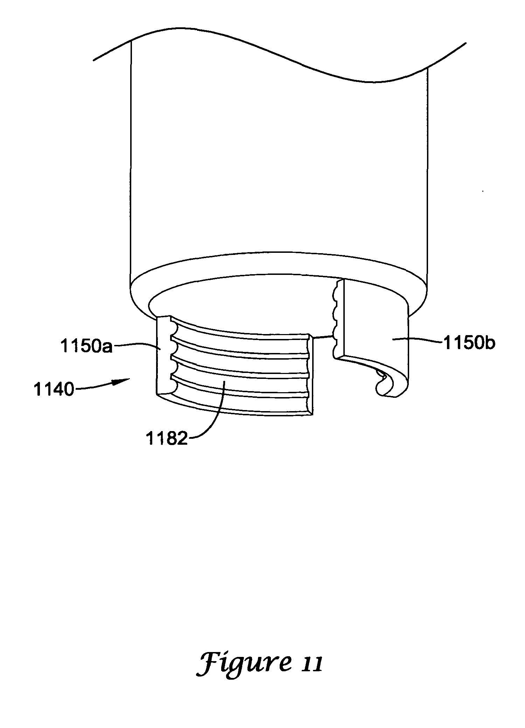

FIG. 11 illustrates another example securing member 1140 that may be similar in form and function to other securing members disclosed herein. Securing member 1140 may include a plurality of tabs 1150a/1150b having threads 1182 formed thereon. Other than having threads 1182 formed thereon, tabs 1150a/1150b may be similar to tabs 150a/150b, tabs 250a/250b, and/or tabs 750a/750b. Securing member 1140 may be configured to attach to an access port by threading onto the port (e.g., port 20 or a version of port 20 with corresponding threads).

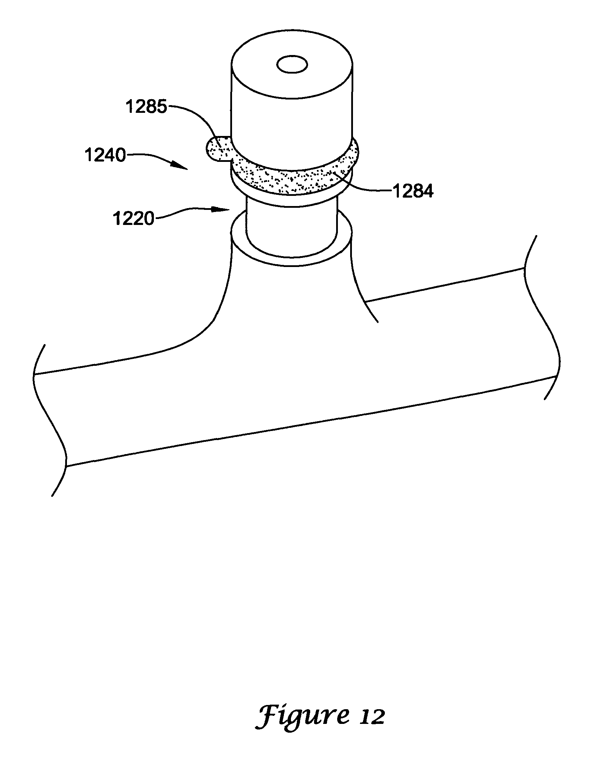

FIG. 12 illustrates another example securing member 1240 that may be similar in form and function to other securing members disclosed herein. Securing member 1240 may include a "quick release" pull tab 1284. Tab 1284 may be disposed on the end of port 1220. In some embodiments, tab 1284 may include an adhesive that is configured to form a seal at port 1220 and thus hold the biopsy cap onto port 1220. In addition, the adhesive in tab 1284 may be disrupted or pulled thereon, for example at a pull point 1285, so that the cap can be quickly and easily removed from port 1220, as desired.

In addition to those securing members disclosed herein, any of the caps disclosed herein may alternatively include other suitable securing members without departing from the spirit of the invention. For example, other securing members may include, but are not intended to be limited to, lock and key designs, slidable or depressible button locks that may catch or otherwise secure onto the port, long sleeves that are disposed over the port, fasteners that includes adhesives including pressure-sensitive adhesives (e.g., that may be analogous to those used on canning lids), and the like. The same may also be true of the various locking members disclosed below.

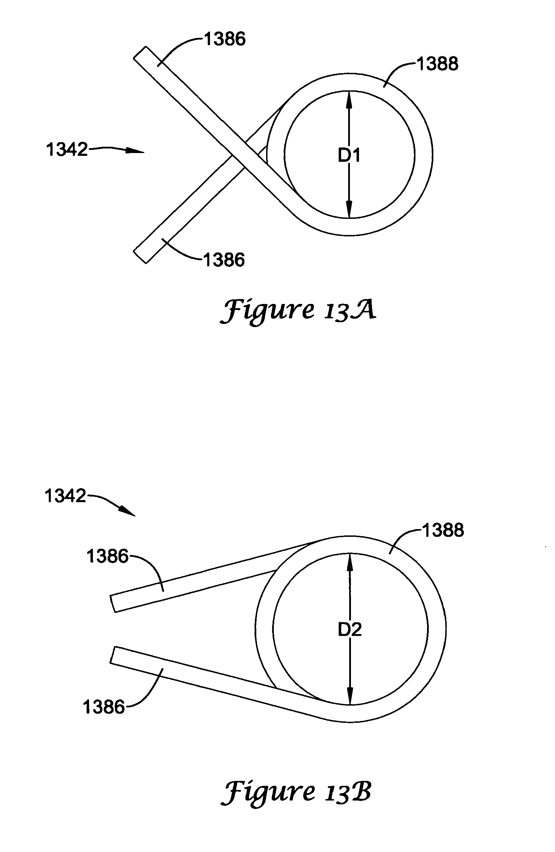

FIGS. 13A-18 illustrate example locking members that may be utilized with any of the biopsy caps disclosed herein. These locking members may be attached to a biopsy cap at any suitable position thereon and they may be used to secure the position of a medical device (e.g., a guidewire, catheter, etc.) relative to the cap (and/or endoscope 10). Just like was the case for the securing members disclosed above, some of the additional cap structure is omitted from FIGS. 13A-18 for simplicity purposes. However, it can be appreciated that any of the locking members shown and contemplated may be attached to a biopsy cap using conventional methods to achieve the desired result.

FIGS. 13A and 13B illustrate locking member 1342, which may be configured to shift between a first configuration (as illustrated in FIG. 13A) and a second configuration (as illustrated in FIG. 13B). Locking member 1342 may include a pair of actuating arms 1386 that, when actuated, shift a locking ring 1388 from the first or smaller configuration that defines a smaller diameter D1 to the second or larger configuration that defines a larger diameter D2. Locking member 1342 may be described as being a spring clip or spring wing as locking ring 1388 may include a plurality of loops of material with a spring-like configuration. The extra portion or loops of the "spring" may be utilized to accommodate the expansion in size of ring 1388. In at least some embodiments, locking member 1342 may have a form similar to a clip that may be used to secure weights onto a barbell.

Although not shown, locking member 1342 may be attached to a biopsy cap at any suitable location using any suitable means. For example, a portion of arms 1386 and/or ring 1388 may be directly attached to a cap. Alternatively, an arm or member may extend from the cap that attaches to locking member 1342. In still other embodiments, locking member 1342 may include an additional structure such as a clip to removably secure locking member 1342 to a cap. These later embodiments of locking member 1342 and other locking members may be desirable because they may allow different types of locking members to be "mixed and matched" based on their particular applicability to a given intervention.

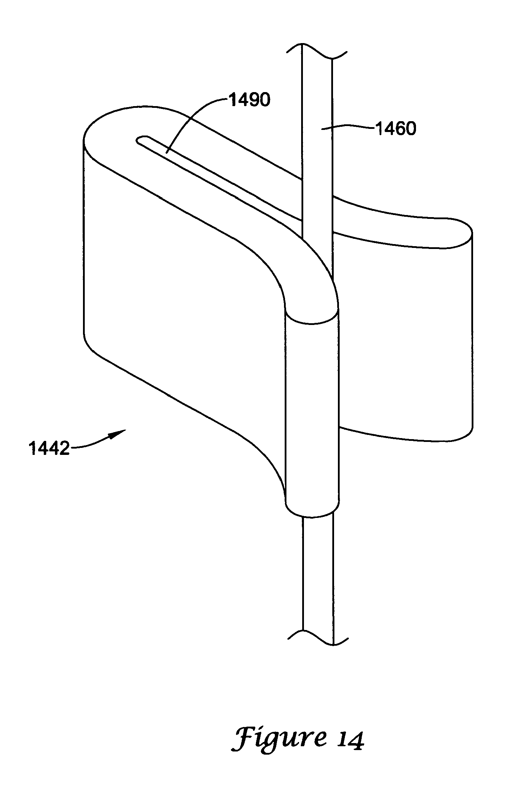

FIG. 14 illustrates another example locking member 1442, which may be used with any of the biopsy caps disclosed herein. Locking member 1442 may have a wedge-like shape and may have a channel or groove 1490 formed therein where device 1460 (e.g., a guidewire, catheter, etc.) can be disposed therein and held by friction. Just like the other locking members disclosed herein, locking member 1442 may be attached to a biopsy cap at any suitable location using any suitable means.

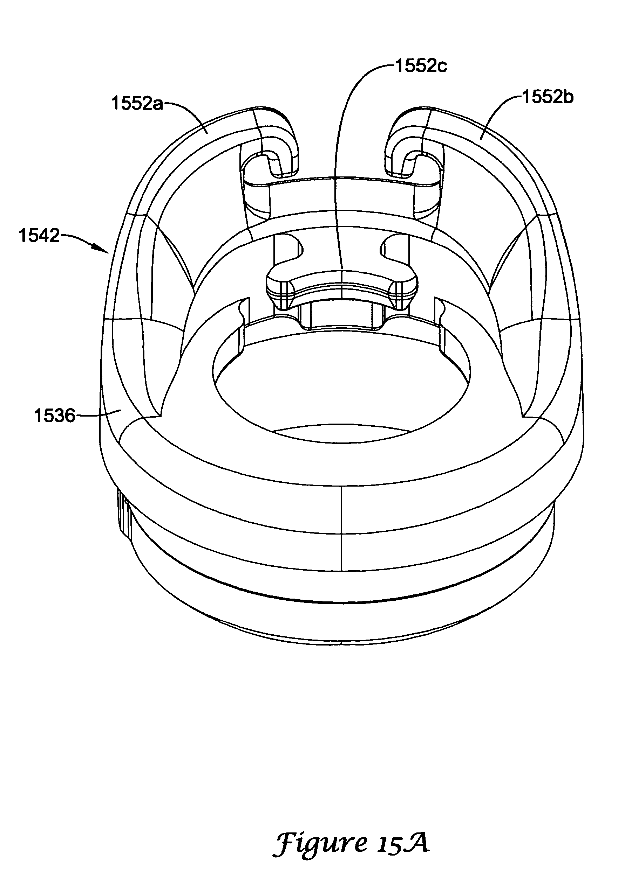

FIG. 15A illustrates another example locking member or locking assembly 1542, which may be used with any of the biopsy caps disclosed herein. Locking member 1542 may include a plurality of locking features including, for example, a pair of arms 1552a/1552b that are coupled to or integrally formed on shell 1536. Arms 1552a/1552b may be shaped in a manner that may allow them to secure the position of a device (e.g., a guidewire, catheter, etc.). For example, arms 1552a/1552b may include one or more bends, hooks, grooves, and/or the like. Locking member 1542 may also include another locking structure or arm 1552c that may be disposed below arms 1552a/1552b. By virtue of having this position, arm 1552c may be used in conjunction with one or more of arms 1552a/1552b to allow the device to be wrapped around the desired combination of structures 1552a/1552b/1552c.

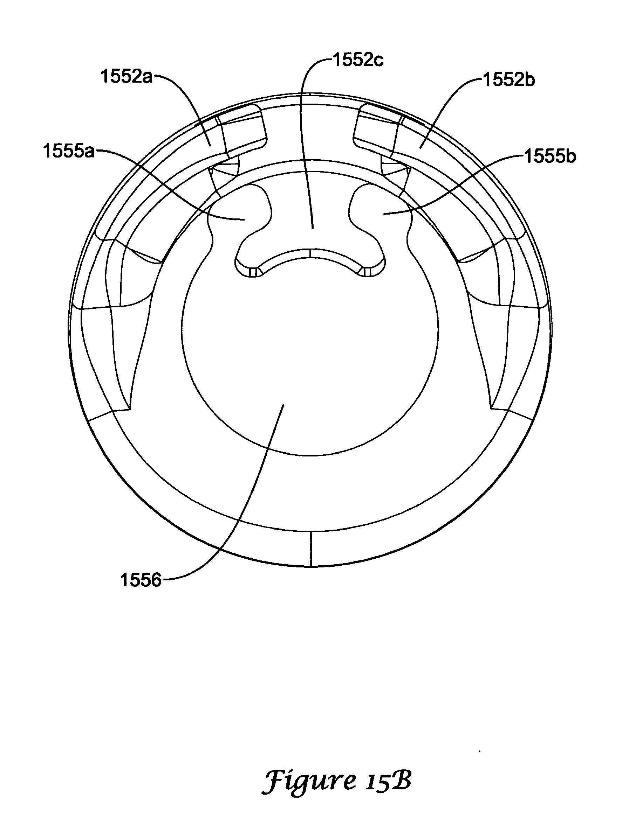

As illustrated in FIG. 15B, which is a rotated view of the locking member 1542 of FIG. 15A, the arm 1552c is shaped to create slotted openings 1555a/1555b in cooperation with the opening 1556 in the upper end of the shell. In some embodiments, the slotted opening is shaped with a narrowed opening which expands into a larger instrument holding area that has contoured surfaces for easy placement and removal of an instrument.

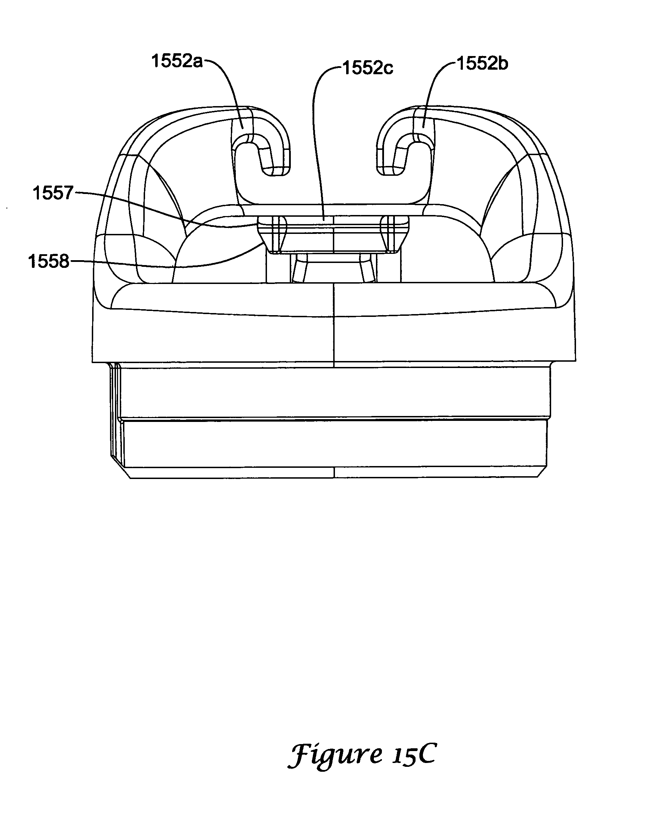

FIG. 15C provides further detail of an exemplary design of arm 1552c. As indicated, the surface of arm 1552c is contoured to provide easy movement of a guidewire or instrument around its surface. Further, the edge 1557 includes an open shoulder 1558 along the lower portion of the lateral surface of arm 1552c. This surface helps prevent instruments from catching on arm 1552c.

As illustrated in FIGS. 15A-15C, the locking member of this illustrative embodiment creates three points of contact with an instrument secured therethrough, such as a guidewire. First, the locking member includes at least one arm extending radially inward from the outer shell to provide the first point of contact with the instrument secured therethrough. Second, the locking member outer shell itself provides a second point of contact with the instrument secured therethrough. Third, the locking member includes a hook assembly extending from the outer shell to provide a third point of contact with the instrument secured therethrough. In the illustrated design, the hook assembly extends longitudinally from the outer shell with a hook portion formed circumferentially therefrom.

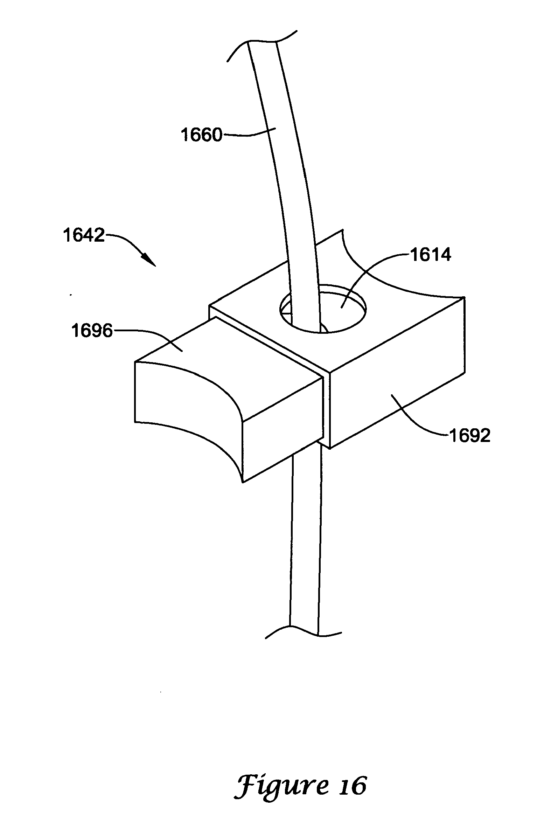

FIG. 16 illustrates another example locking member 1642, which may be used with any of the biopsy caps disclosed herein. Locking member 1642 may include a base 1692 having an opening 1614 formed therein. Device 1660 may extend through opening 1614. A spring button 1696 may be attached to base 1692. Spring button 1696 may be coupled to a spring (not shown) that biases a portion of button 1696 (e.g., a rear portion of button 1696 that may be disposed within base 1692 on the opposite side of opening 1614) into opening 1614, thereby "closing" or "locking" opening 1614. Depressing button 1696 may overcome the bias and open opening 1614 so that device 1660 can be extended therethrough. Releasing button 1696 allows the spring to press button 1696 back into the biased position and lock the position of device 1660.

A number of different configurations are contemplated for locking member 1642. For example, locking member 1642 may have a barrel-like or cylindrical shape rather than the more squared or rectangular shape as shown. In addition, locking member 1642 may include a lock that can reversibly hold button 1696 in the desired position such as, for example, the locked position.

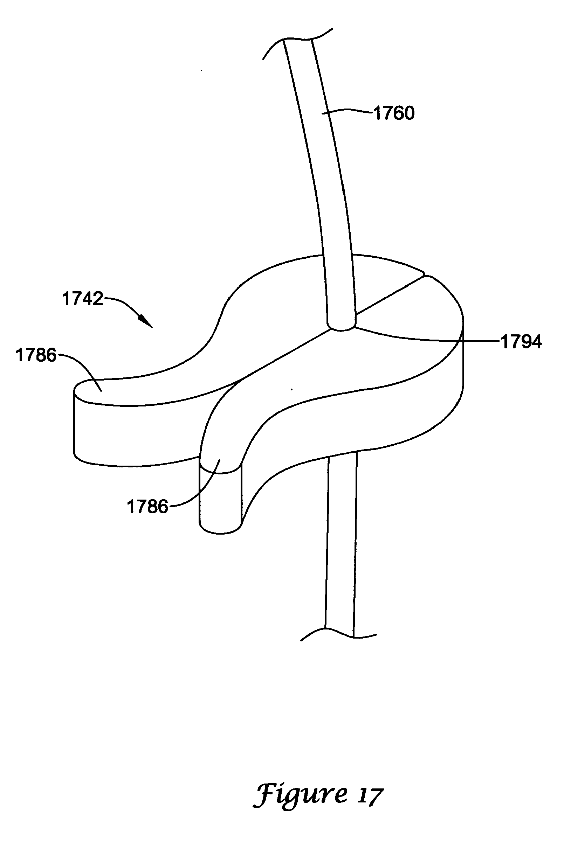

FIG. 17 illustrates another example locking member 1742, which may be used with any of the biopsy caps disclosed herein. Locking member 1742 may include a pair of arms 1786 that can be actuated to open/close opening 1794 to secure device 1760. Locking member 1742 may function in a manner similar to a clothespin. As such, locking member 1742 may include a spring or other biasing member (not shown) that holds it in either the open (e.g., "unlocked") or closed (e.g., "locked") positions.

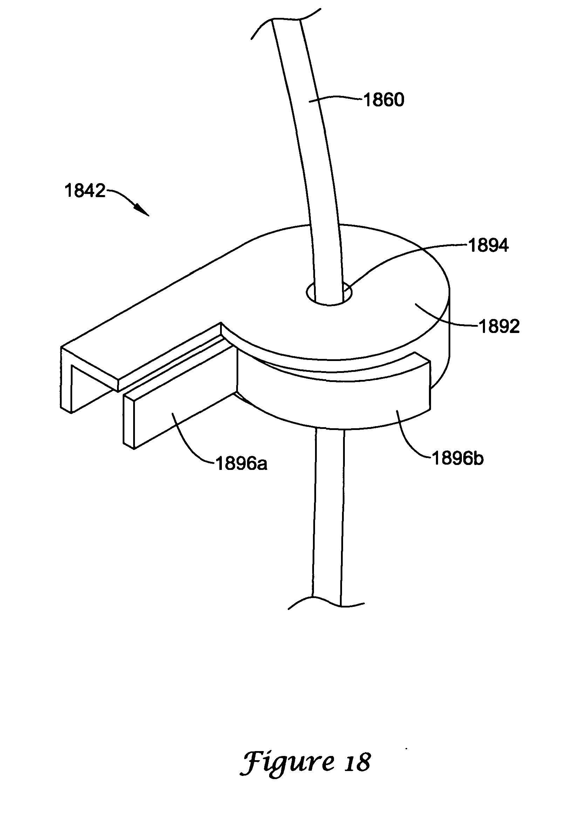

FIG. 18 illustrates another example locking member 1842, which may be used with any of the biopsy caps disclosed herein. Locking member 1842 may include a base 1892 having opening 1894 formed therein. Device 1860 may extend therethrough. A pair of buttons 1896a/1896b may be attached to base 1892 for opening/closing opening 1894. For example, one of the buttons (e.g., button 1896b) may be depressed to "lock" device 1860 while the other button (e.g., button 1896a) may be depressed to open or "unlock" device 1860.

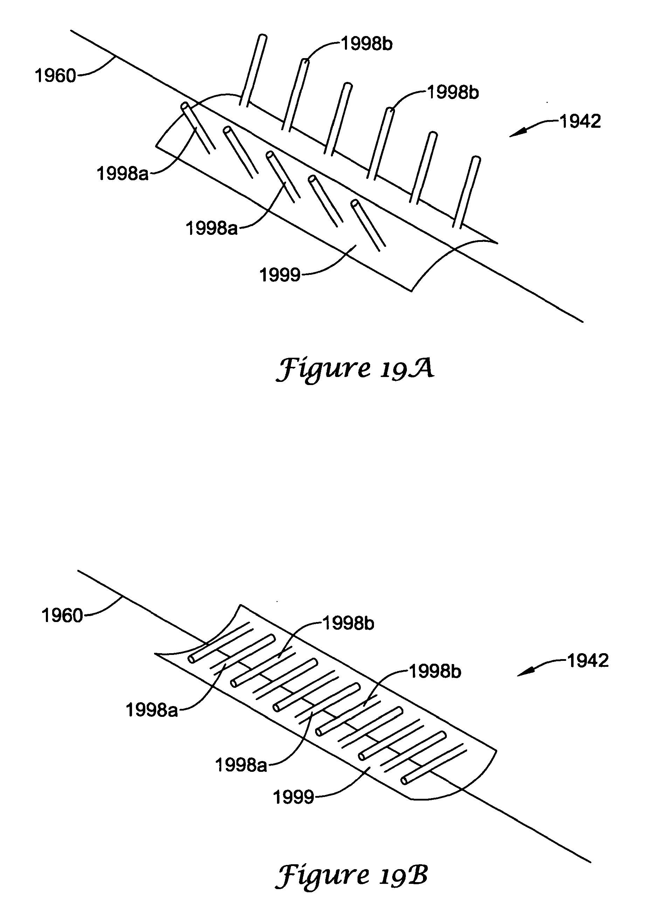

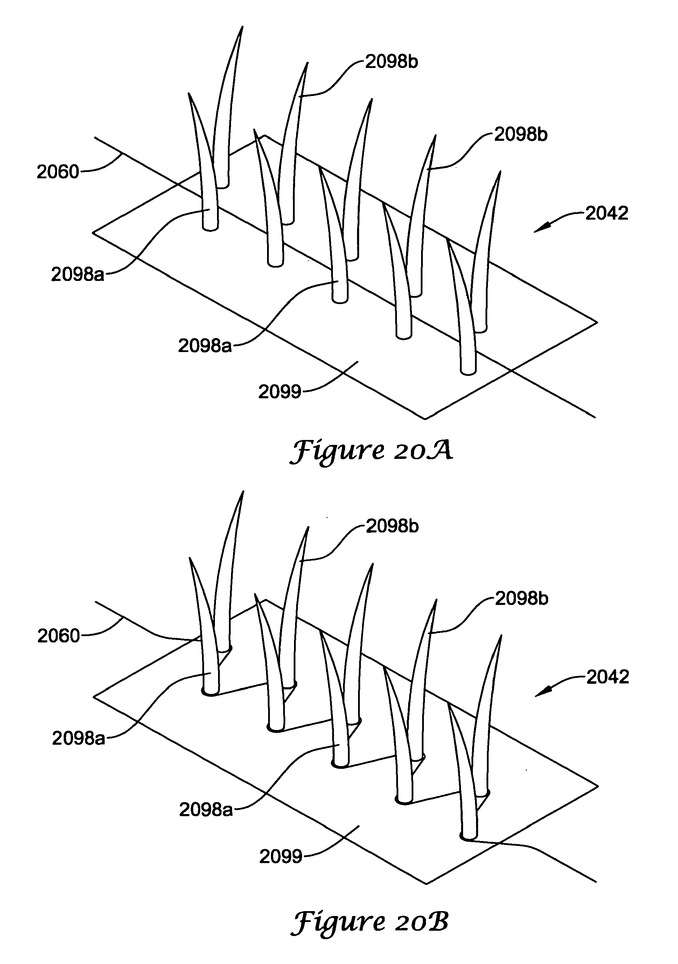

FIGS. 19A and 19B illustrate locking member 1942, which may be configured to shift between a first or open configuration (as illustrated in FIG. 19A) and a second or closed configuration (as illustrated in FIG. 19B). Locking member 1942 may include a pair of opposing sets of fingers 1998a/1998b coupled to a base 1999 that are configured to shift from the upright or open first position to the horizontal or flat second configuration, the later being configured to secure the position of device 1960. FIGS. 20A and 20B illustrate locking member 2042, which may be similar in form and function to locking member 1942. Locking member 2042 may include a pair of opposing sets of fingers 2098a/2098b coupled to a base 2099. Device 2060 may extend through fingers 2098a/2098b as shown in FIG. 20A, which may hold device 2060 in place, for example, by friction. Alternatively, device 2060 may be wrapped around fingers 2098a/2098b, as shown in FIG. 20B.

In some embodiments, bases 1999 and/or 2099 may be generally planar. In other embodiments, bases 1999 and/or 2099 may be curved so as to be convex, concave, or have another shape. Moreover, bases 1999 and/or 2099 may change from one shape to another upon actuation of fingers 1998a/1999b and/or 2098a/2098b. For example, bases 1999 and/or 2099 may be generally planar when fingers 1998a/1999b and/or 2098a/2098b are in the open position and bases 1999 and/or 2099 may shift to a concave shape when fingers 1998a/1999b and/or 2098a/2098b shift to the flat configuration. Alternatively, bases 1999 and/or 2099 may shift from concave to planar, convex to planar, planar to convex, etc.

A number of alternatives are also contemplated for fingers 1998a/1999b and/or 2098a/2098b. For example, fingers 1998a/1999b and/or 2098a/2098b may be interconnected so that the shifting of one finger results in the shifting of all the fingers. Alternatively, flaps may be used instead of or in addition to fingers 1998a/1999b and/or 2098a/2098b that extend down at least a portion of the length of bases 1999 and/or 2099 and that are configured to shift between an open and a closed configuration.

Base 1999/2099 of locking members 1942/2042 may desirably add a surface substrate that may allow these devices to be attached to a biopsy cap. In some embodiments, base 1999/2099 may include a strip of polymer or plastic that can be bonded to a biopsy cap with a permanent adhesive. In other embodiments, base 1999/2099 may be configured to be removably attached to the biopsy cap. For example, a removable or temporary adhesive may be used, base 1999/2099 may be "velcroed" onto the cap, etc.

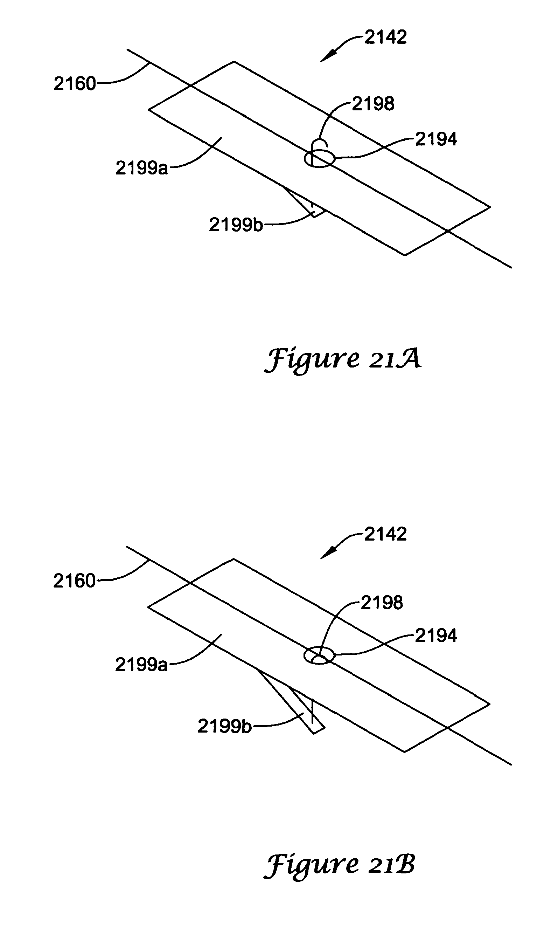

FIGS. 21A and 21B illustrate locking member 2142, which may be configured to shift between a first configuration (as illustrated in FIG. 21A) and a second configuration (as illustrated in FIG. 21B). Locking member 2142 may include a base 2199a including a platform region 2199b. Region 2199b may include a hook-like extension 2198 that extends through an opening 2194 in base 2199a and that can grasp device 2160 when actuated (as illustrated in FIG. 21A). Region 2199b may be hingedly connected to base 2199a so that region 2199b can be moved up or down, as desired, to engage device 2160. In alternative embodiments, multiple hook-like extensions 2198 may be utilized. Furthermore, hook-like extensions 2198 having different shapes may also be utilized such as longer hooks, wider hooks, two or more opposing hooks, eyelets, etc.

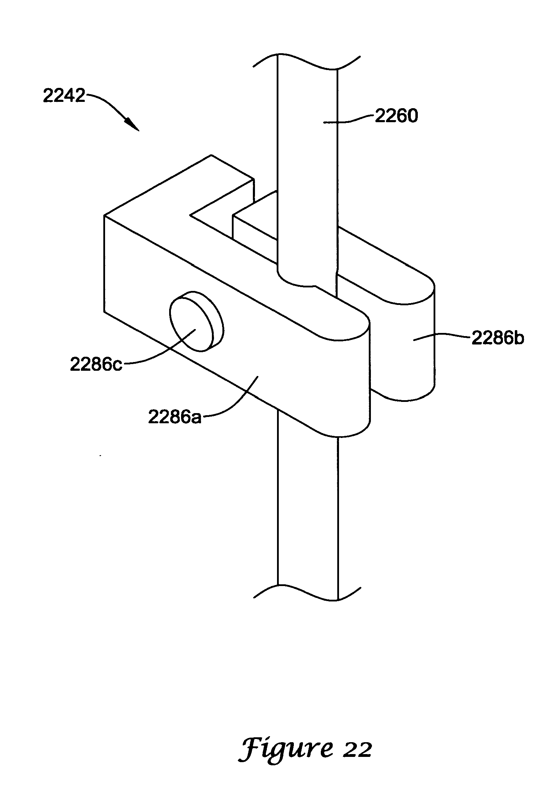

FIG. 22 illustrates another example locking device 2260, which may be used with any of the biopsy caps disclosed herein. Device 2260 may include a pair of arm segments 2286a/2286b coupled together by a linkage 2286c. Linkage 2286c may be slidable within one of the arm segments 2286a/2286b so that arms 2286a/2286b can be brought into closer contact with one another by pinching together arms 2286a/2286b and locking the position of device 2260. Manually moving arms 2286a/2286b further apart may release device 2260.

In some embodiments, one or more additional locking members may be added to a cap. The additional locking member may take any suitable form including any of those disclosed herein. Adding the locking members may include fastening, snapping on, or hingedly connecting an external locking member assembly onto the cap. Some additional discussion of wire or other locking devices which may be suitable for use with a biopsy cap may include U.S. Patent Application Pub Nos. US20060229496A1, US20050148820A1, and US20040106852A1 as well as U.S. Patent Nos. 7,060,052, 7,037,293, 6,893,393, 6,663,597, and 6,096,009.

The various caps as well as the various components thereof may be manufactured according to essentially any suitable manufacturing technique including molding, casting, mechanical working, and the like, or any other suitable technique. Furthermore, the various structures may include materials commonly associated with medical devices such as metals, metal alloys, polymers, metal-polymer composites, ceramics, combinations thereof, and the like, or any other suitable material. These materials may include transparent or translucent materials to aid in visualization during the procedure. Some examples of suitable metals and metal alloys include stainless steel, such as 304V, 304L, and 316LV stainless steel; mild steel; nickel-titanium alloy such as linear-elastic and/or super-elastic nitinol; other nickel alloys such as nickel-chromium-molybdenum alloys (e.g., UNS: N06625 such as INCONEL® 625, UNS: N06022 such as HASTELLOY® C-22®, UNS: N10276 such as HASTELLOY® C276®, other HASTELLOY® alloys, and the like), nickel-copper alloys (e.g., UNS: N04400 such as MONEL® 400, NICKELVAC® 400, NICORROS® 400, and the like), nickel-cobalt-chromium-molybdenum alloys (e.g., UNS: R30035 such as MP35-N® and the like), nickel-molybdenum alloys (e.g., UNS: N10665 such as HASTELLOY® ALLOY B2®), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R30003 such as ELGILOY®, PHYNOX®, and the like); platinum enriched stainless steel; combinations thereof; and the like; or any other suitable material.

Some examples of suitable polymers may include polytetrafluoroethylene (PTFE), ethylene tetrafluoroethylene (ETFE), fluorinated ethylene propylene (FEP), polyoxymethylene (POM, for example, DELRIN® available from DuPont), polyether block ester, polyurethane, polypropylene (PP), polyvinylchloride (PVC), polyetherester (for example, ARNITEL® available from DSM Engineering Plastics), ether or ester based copolymers (for example, butylene/poly(alkylene ether) phthalate and/or other polyester elastomers such as HYTREL® available from DuPont), polyamide (for example, DURETHAN® available from Bayer or CRISTAMID® available from Elf Atochem), elastomeric polyamides, block polyamide/ethers, polyether block amide (PEBA, for example available under the trade name PEBAX®), ethylene vinyl acetate copolymers (EVA), silicones, polyethylene (PE), Marlex high-density polyethylene, Marlex low-density polyethylene, linear low density polyethylene (for example REXELL®), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR®), polysulfone, nylon, nylon-12 (such as GRILAMID® available from EMS American Grilon), perfluoro(propyl vinyl ether) (PFA), ethylene vinyl alcohol, polyolefin, polystyrene, epoxy, polyvinylidene chloride (PVdC), polycarbonates, ionomers, biocompatible polymers, other suitable materials, or mixtures, combinations, copolymers thereof, polymer/metal composites, and the like.

In at least some embodiments, portions or all of the structures disclosed herein may also be doped with, made of, or otherwise include a radiopaque material. Radiopaque materials are understood to be materials capable of producing a relatively bright image on a fluoroscopy screen or another imaging technique during a medical procedure. This relatively bright image aids the user of endoscope 10 in determining its location. Some examples of radiopaque materials can include, but are not limited to, gold, platinum, palladium, tantalum, tungsten alloy, polymer material loaded with a radiopaque filler, and the like. Additionally, radiopaque marker bands and/or coils may be incorporated into the design of endoscope 10 or the various components thereof to achieve the same result.

In some embodiments, a degree of MRI compatibility may be imparted into the structures disclosed herein. For example, to enhance compatibility with Magnetic Resonance Imaging (MRI) machines, it may be desirable to make a portion of endoscope 10 in a manner that would impart a degree of MRI compatibility. For example, a portion of endoscope 10 may be made of a material that does not substantially distort the image and create substantial artifacts (artifacts are gaps in the image). Certain ferromagnetic materials, for example, may not be suitable because they may create artifacts in an MRI image. A portion of endoscope 10 may also be made from a material that the MRI machine can image. Some materials that exhibit these characteristics include, for example, tungsten, cobalt-chromium-molybdenum alloys (e.g., UNS: R30003 such as ELGILOY®, PHYNOX®, and the like), nickel-cobalt-chromium-molybdenum alloys (e.g., UNS: R30035 such as MP35-N® and the like), nitinol, and the like, and others.

In addition, portions or components of the structures (including the various securing members, locking members, etc.) disclosed herein may be coated with a relatively soft material that may improve grip such as a thermoplastic elastomer. The coating may or may not include additional features that may improve grip such as ridges, surface textures, bumps, grooves, projections, etc.

Furthermore, the various structures disclosed herein may be designed for single use or may be designed for repeated uses. Thus, the structures disclosed herein may be manufactured from materials that can withstand multiple sterilizations and/or cleanings. This may be true of entire caps, as disclosed herein, or any of the various features of any of the caps.

It should be understood that this disclosure is, in many respects, only illustrative. Changes may be made in details, particularly in matters of shape, size, and arrangement of steps without exceeding the scope of the invention. The invention's scope is, of course, defined in the language in which the appended claims are expressed.

## Claims

1. An endoscope biopsy cap (300), comprising:
an outer shell of the biopsy cap having an opening (1556) formed therein and a base;
a securing member (332) disposed on the base for securing the base to a port on an endoscope;
a locking assembly (1542) for securing the position of a medical instrument disposed in the opening, the locking assembly being integral with the outer shell; and
a seal member (350) disposed within the outer shell,
wherein the locking assembly includes three points of contact with the instrument secured therethrough;
wherein the locking assembly includes at least one arm (324, 1552c) extending radially inward from the outer shell to provide a first point of contact with the instrument secured therethrough;
wherein the outer shell provides a second point of contact with the instrument secured therethrough; and
wherein the locking assembly includes a hook assembly (326, 1552a, 1442b) extending from the outer shell to provide a third point of contact with the instrument secured therethrough.

2. The cap of claim 1, wherein the at least one arm is a pair of arms coupled together by a linkage.

3. The cap of claim 1, wherein the hook assembly extends longitudinally from the outer shell with a hook portion formed circumferentially therefrom.

4. The cap of claim 1, wherein the seal member includes an upper seal, a lower seal and a fluid control region defined therebetween.

5. The cap of claim 4, wherein the fluid control region is defined by an area of vacant space within the seal member.

6. The cap of claim 5, further comprising a foam seal member disposed in the fluid control region.

7. The cap of claim 4, wherein the fluid control region includes an absorbent gel.

8. The cap of claim 1, wherein the securing member comprises a mouth formed in the base for lateral disposition of a port on an endoscope.

9. The cap of claim 8, wherein the mouth includes a lower ramped portion for guiding the port into the cap.

10. The cap of claim 8, wherein a bottom portion of the seal member is disposed in the mouth for sealing engagement with a port on an endoscope.

## Patentansprüche

1. Endoskopbiopsiekappe (300), die aufweist:
eine äußere Hülle der Biopsiekappe mit einer darin ausgebildeten Öffnung (1556) und einer Basis;
ein Sicherungselement (332), das an der Basis zur Sicherung der Basis an einem Zugang an einem Endoskop angeordnet ist;
eine Verriegelungsanordnung (1542) zur Sicherung der Position eines medizinischen Instruments, das in der Öffnung angeordnet ist, wobei die Verriegelungsanordnung integral mit der äußeren Hülle ist; und
ein Dichtungselement (350), das in der äußeren Hülle angeordnet ist,
wobei die Verriegelungsanordnung drei Berührungspunkte mit dem dadurch gesicherten Instrument aufweist;
wobei die Verriegelungsanordnung mindestens einen Arm (324, 1552c) aufweist, der sich von der äußeren Hülle radial nach innen erstreckt, um einen ersten Berührungspunkt mit dem dadurch gesicherten Instrument bereitzustellen;
wobei die äußere Hülle einen zweiten Berührungspunkt mit dem dadurch gesicherten Instrument bereitstellt; und
wobei die Verriegelungsanordnung eine Hakenanordnung (326, 1552a, 1442b) aufweist, die sich von der äußeren Hülle erstreckt, um einen dritten Berührungspunkt mit dem dadurch gesicherten Instrument bereitzustellen.

2. Kappe nach Anspruch 1, wobei der mindestens eine Arm aus einem Armpaar besteht, das durch eine Verbindung miteinander gekoppelt ist.

3. Kappe nach Anspruch 1, wobei sich die Hakenanordnung mit einem in Umfangsrichtung davon ausgebildeten Hakenabschnitt longitudinal von der äußeren Hülle erstreckt.

4. Kappe nach Anspruch 1, wobei das Dichtungselement eine obere Dichtung, eine untere Dichtung und einen dazwischen definierten Fluidkontrollbereich aufweist.

5. Kappe nach Anspruch 4, wobei der Fluidkontrollbereich durch einen Freiraumbereich im Dichtungselement definiert ist.

6. Kappe nach Anspruch 5, die ferner ein Schaumstoffdichtungselement aufweist, das im Fluidkontrollbereich angeordnet ist.

7. Kappe nach Anspruch 4, wobei der Fluidkontrollbereich ein Absorptionsgel aufweist.

8. Kappe nach Anspruch 1, wobei das Sicherungselement eine Mündung aufweist, die in der Basis zur lateralen Anordnung eines Zugangs an einem Endoskop angeordnet ist.

9. Kappe nach Anspruch 8, wobei die Mündung einen unteren geneigten Abschnitt zur Führung des Zugangs in die Kappe aufweist.

10. Kappe nach Anspruch 8, wobei ein unterer Abschnitt des Dichtungselements zum dichtenden Eingriff mit einem Zugang an einem Endoskop in der Mündung angeordnet ist.

## Revendications

1. Calotte (300) d'endoscope pour biopsie, comprenant :
une coque extérieure de la calotte pour biopsie avec un orifice (1556) ménagé dans celle-ci et une base ; un élément de fixation (332) disposé sur la base pour fixer ladite base à un point de connexion sur un endoscope ;
un ensemble de verrouillage (1542) pour fixer la position d'un instrument médical disposé dans l'orifice, ledit ensemble de verrouillage étant intégré à la coque extérieure ; et
un élément d'étanchéité (350) disposé à l'intérieur de la coque extérieure,
l'ensemble de verrouillage présentant trois points de contact avec l'instrument fixé dans celui-ci ;
l'ensemble de verrouillage comportant au moins un bras (324, 1552c) s'étendant radialement vers l'intérieur depuis la coque extérieure, pour réaliser un premier point de contact avec l'instrument fixé dans celle-ci ;
la coque extérieure réalisant un deuxième point de contact avec instrument fixé dans celle-ci ; et
l'ensemble de verrouillage comportant un dispositif à crochet (326, 1552a, 1442b) s'étendant depuis la coque extérieure pour réaliser un troisième point de contact avec l'instrument fixé dans celui-ci.

2. Calotte selon la revendication 1, où le ou les bras consistent en une paire de bras accouplés l'un à l'autre par une pièce de liaison.

3. Calotte selon la revendication 1, où le dispositif à crochet s'étend longitudinalement depuis la coque extérieure avec une partie de crochet formée sur la circonférence de celle-ci.

4. Calotte selon la revendication 1, où l'élément d'étanchéité comporte un joint supérieur, un joint inférieur et une zone de régulation de fluide définie entre les deux.

5. Calotte selon la revendication 4, où la zone de régulation de fluide est définie par une zone d'espace libre à l'intérieur de l'élément d'étanchéité.

6. Calotte selon la revendication 5, comprenant en outre un élément d'étanchéité en mousse disposé dans la zone de régulation de fluide.

7. Calotte selon la revendication 4, où la zone de régulation de fluide contient un gel absorbant.

8. Calotte selon la revendication 1, où l'élément de fixation comprend une embouchure formée dans la base pour la mise en place latérale d'un point de connexion sur un endoscope.

9. Calotte selon la revendication 8, où l'embouchure comporte une partie inférieure avec une inclinaison pour le guidage du point de connexion dans la calotte.

10. Calotte selon la revendication 8, où une partie de fond de l'élément d'étanchéité est disposé dans l'embouchure pour rendre étanche la connexion avec un point de connexion sur un endoscope.
